(19) Europäisches Patentamt
European Patent Office
Office européen des brevets

(11) Veröffentlichungsnummer: **0 420 807 A1**

# (12) EUROPÄISCHE PATENTANMELDUNG

(21) Anmeldenummer: **90810713.9**

(22) Anmeldetag: **18.09.90**

(51) Int. Cl.5: **C08G 65/32**, C08G 65/26, D06P 1/613

(30) Priorität: **27.09.89 CH 3496/89**

(43) Veröffentlichungstag der Anmeldung: **03.04.91 Patentblatt 91/14**

(84) Benannte Vertragsstaaten: **CH DE FR GB IT LI**

(71) Anmelder: **CIBA-GEIGY AG**
**Klybeckstrasse 141**
**CH-4002 Basel(CH)**

(72) Erfinder: **Töpfl, Rosemarie**
**Dorneckstrasse 68**
**CH-4143 Dornach(CH)**

## (54) Anlagerungsprodukte von Alkylenoxid und Styroloxid an Arylalkanole.

(57) Anlagerungsprodukte eines Alkylenoxids und Styroloxid an ein Arylalkanol, insbesondere Anlagerungsprodukte der Formeln

(1) 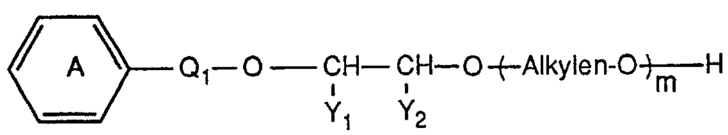

(2) 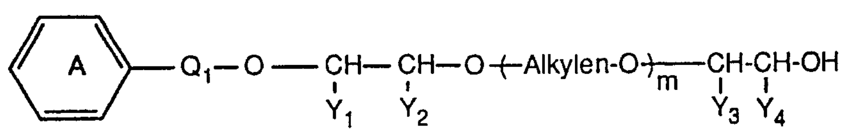

(3) 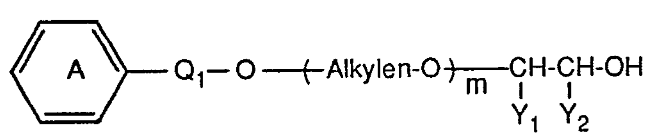

und ihre sauren Ester und deren Salze, in denen der Ring A einen unsubstituierten oder einen durch Halogen, Methyl oder $C_1$-$C_3$-Alkoxy substituierten Phenylrest,
$Q_1$ Alkylen mit 1 bis 4 Kohlenstoffatomen, besonders Methylen,
in den Substituentenpaaren ($Y_1$ und $Y_2$) und ($Y_3$ und $Y_4$) ein Y Phenyl und das andere Y Wasserstoff,
"Alkylen" einen Alkylenrest von 2 oder 3 Kohlenstoffatomen und m eine Zahl von 1 bis 80 bedeuten.

Diese Produkte stellen eine neue Klasse von nichtionogenen oder anionischen Tensiden, welche als Textilveredlungsmittel verwendet werden.

EP 0 420 807 A1

# ANLAGERUNGSPRODUKTE VON ALKYLENOXID UND STYROLOXID AN ARYLALKANOLE

Die vorliegende Erfindung betrifft Anlagerungsprodukte von Alkylenoxid und Styroloxid an Arylalkanole sowie ihre Verwendung, insbesondere als Emulgiermittel, Färbereihilfsmittel und besonders als Netzmittel oder Foulardierhilfsmittel.

Die erfindungsgemässen Produkte sind dadurch gekennzeichnet, dass sie durch Anlagerung eines Alkylenoxides und Styroloxid an ein Arylalkanol erhalten werden und auch in Form ihrer sauren Ester und deren Salze hergestellt werden.

Die Anlagerung kann in beliebiger Reihenfolge durchgeführt werden, wobei 1 bis 100 Alkylenoxid und 1 oder 2 Mol Styroloxid eingesetzt werden können.

Als Arylalkanol kann beispielsweise Benzylalkohol, α-Methylbenzylalkohol, 2-Phenylethanol oder Phenylisopropanol verwendet werden. Bevorzugt ist Benzylalkohol.

Als Alkylenoxid können Ethylenoxid, Propylenoxid oder Butylenoxid in Betracht kommen.

Bevorzugt sind Anlagerungsprodukte der Formel

(1) 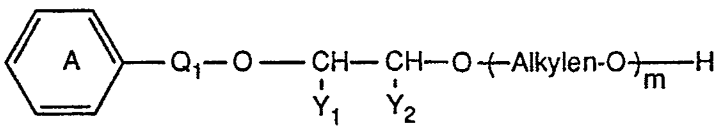

und ihre sauren Ester und deren Salze.

Weitere Anlagerungsprodukte entsprechen den Formeln

(2) 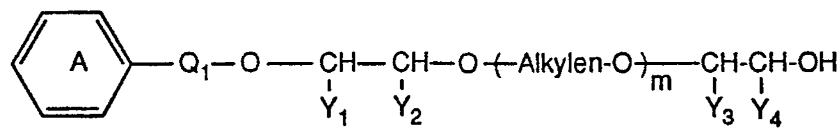 und

(3) 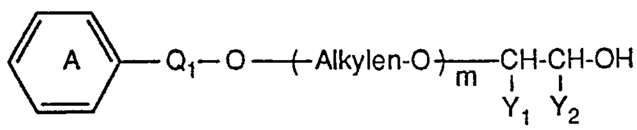

Auch diese Produkte der Formeln (2) und (3) können in Form ihrer sauren Ester und deren Salze vorliegen.

In den Formeln (1), (2) und (3) bedeutet der Ring A einen unsubstituierten oder einen durch Halogen, Methyl oder $C_1$-$C_3$-Alkoxy, wie Isopropoxy und besonders Methoxy substituierten Phenylrest.

$Q_1$ bedeutet Alkylen mit 1 bis 4 Kohlenstoffatomen, vorzugsweise Ethylen oder besonders Methylen.

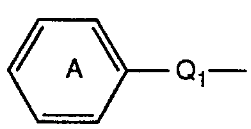

stellt vorteilhafterweise den Kohlenwasserstoffrest eines araliphatischen Alkohols mit 7 bis 9 Kohlenstoffatomen dar. Vorzugsweise bedeutet

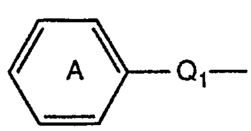

den Benzylrest.

Als araliphatische Alkohole können z.B. Benzylalkohol, Phenylethanol, Methylphenylethanol, Chlorphenylethanol oder Phenylisopropanol in Betracht kommen.

"Alkylen" steht für einen Alkylenrest von 2 oder 3 Kohlenstoffatomen, wie Ethylen oder Propylen oder deren Kombinationen. m bedeutet eine Zahl von 1 bis 80, vorzugsweise 2 bis 40 und insbesondere 2 bis 10.

(Alkylen-O)Ketten sind bevorzugt vom Ethylenglykol-, Propylenethylenglykol- oder Ethylenpropylenglykol-Typus; besonders bevorzugt ist erstere.

In den Substituentenpaaren ($Y_1$ und $Y_2$) und ($Y_3$ und $Y_4$) bedeutet ein Y Phenyl und das andere Y Wasserstoff. Vorzugsweise sind $Y_1$ und $Y_3$ Phenyl und $Y_2$ und $Y_4$ Wasserstoff.

Die Anlagerungsprodukte der Formel (1) werden hergestellt, indem man zuerst den araliphatischen Alkohol mit 1 Mol Styroloxid verethert und dann mit 1 bis 80 Mol Alkylenoxid (Ethylenoxid und/oder Propylenoxid) umsetzt.

Die Veretherung des araliphatischen Alkohols mit Styroloxid erfolgt zweckmässigerweise in einem praktisch wasserfreien oder wasserarmen Medium bei einer Temperatur von 40 bis 90 °C mit oder ohne Druck und in Gegenwart von Bortrifluoridethyletheral.

Die Anlagerung des Alkylenoxids an das substituierte Phenylethanol wird nach an sich bekannten Methoden durchgeführt, wobei Ethylenoxid oder Propylenoxid oder abwechslungsweise Ethylenoxid und Propylenoxid oder Mischungen von Ethylenoxid und Propylenoxid verwendet werden. Die Anlagerungsprodukte der Formel (2) werden hergestellt, indem man die Produkte der Formel (1) endständig mit einem weiteren Mol Styroloxid verethert.

Die Anlagerungsprodukte der Formel (3) werden hergestellt, indem man zuerst den araliphatischen Alkohol mit 1 bis 80 Mol Alkylenoxid (Ethylenoxid und/oder Propylenoxid) verethert und dann 1 Mol Styroloxid an den erhaltenen Polyalkylenglykolether anlagert.

Sowohl die Veretherung des araliphatischen Alkohols als auch die endständige Styroloxidanlagerung können nach den obengenannten Bedingungen durchgeführt werden.

Die sauren Ester können je nach dem Säurerest in Form von Monoester, Diester oder Halbester und als freie Säuren oder vorzugsweise als Salze z.B. Alkalimetall- oder Erdalkalimetallsalze oder Ammoniumsalze vorliegen. Als Alkalimetallsalze seien insbesondere die Natrium-, Kalium- oder Lithiumsalze, als Erdalkalimetallsalze die Magnesium- und Calciumsalze und als Ammoniumsalze die Ammonium-, Dimethylammonium-, Trimethylammonium-, Monoethanolammonium-, Diethanolammonium- und Triethanolammoniumsalze genannt. Vorzugsweise werden die sauren Ester als Ammoniumsalze hergestellt. Mono- oder Diethanolammoniumsalze können noch weiter mit 1 bis 4 Oxyethylen-Einheiten verethert sein.

Die sauren Ester werden hergestellt, indem man das erfindungsgemässe nichtionogene Anlagerungsprodukt z.B. der Formel (1), (2) oder (3) mit einer mindestens zweibasischen Sauerstoffsäure umsetzt und gewünschtenfalls den erhaltenen sauren Ester in die obengenannten Salze überführt.

Als mehrbasische Sauerstoffsäuren für die Bildung der sauren Ester können gegebenenfalls sulfonierte, organische, vorzugsweise aliphatische Dicarbonsäuren von 3 bis 6 Kohlenstoffatomen, wie z.B. Maleinsäure, Malonsäure, Bernsteinsäure oder Sulfobernsteinsäure, oder mehrbasische anorganische Sauerstoffsäuren, wie z.B. Schwefelsäure oder Orthophosphorsäure dienen. Anstelle der Säuren können deren funktionelle Derivate wie Säureanhydride, Säurehalogenide oder Säureamide verwendet werden. Als Beispiele dieser funktionellen Derivate seien Maleinsäureanhydrid, Phosphorpentoxid, Chlorsulfonsäure und Sulfaminsäure genannt. Die Phosphorsäureester entstehen zweckmässigerweise als Gemische eines Mono-und Diesters.

Die Veresterung wird in der Regel durch einfaches Vermischen der Reaktionspartner unter Erwärmen, zweckmässig auf eine Temperatur zwischen 50 °C und 100 °C, durchgeführt. Die zunächst entstehenden, freien Säuren können anschliessend in die entsprechenden Alkalimetall- oder Ammoniumsalze übergeführt werden. Die Ueberführung in die Salze erfolgt auf übliche Weise durch Zugabe von Basen, wie z.B. Ammoniak, Monoethanolamin, Triethanolamin oder Alkalimetallhydroxyde, z.B. Natrium- oder Kaliumhydroxyd. Gemäss einer besonders bevorzugten Ausführungsart werden saure Schwefelsäureester in Form ihrer Ammoniumsalze direkt hergestellt, indem man die nichtionogenen Anlagerungsprodukte, zweckmässig in Gegenwart von Harnstoff, mit Sulfaminsäure erwärmt.

Praktisch wichtige Anlagerungsprodukte entsprechen der Formel

(4) 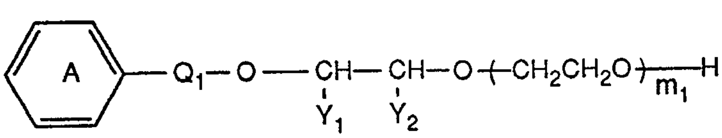

worin von $Y_1$ und $Y_2$ eines Phenyl und das andere Wasserstoff und $m_1$ 2 bis 40 insbesondere 2 bis 10 bedeuten und A und $Q_1$ die angegebenen Bedeutungen haben.

Bevorzugte mit einer anorganischen oder organischen Säure hergestellte saure Ester entsprechen der Formel

(5) 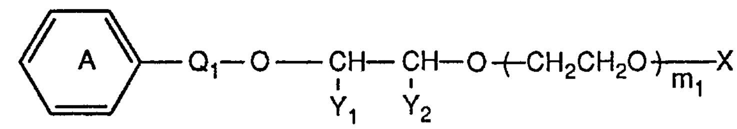

oder der Formel

(6) 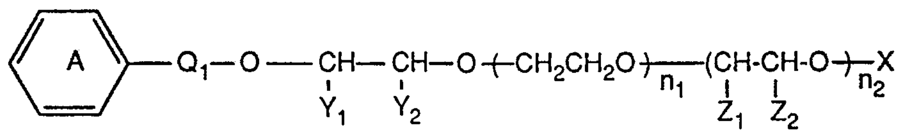

oder der Formel

(7) 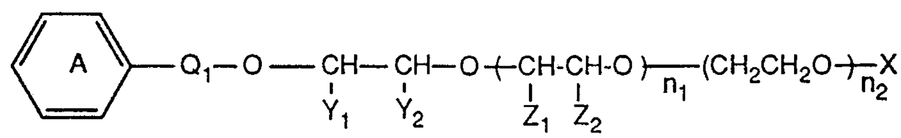

worin A, $Q_1$, $Y_1$, $Y_2$ und $m_1$ die angegebene Bedeutung haben, von $Z_1$ und $Z_2$ eines Methyl und das andere Wasserstoff, X den Maleinsäure-, Sulfobernsteinsäure-, Schwefelsäure- oder Phosphorsäurerest und die Summe von $n_1 + n_2$ 2 bis 30, vorzugsweise 4 bis 18 bedeuten.

Bevorzugte saure Ester der Formeln (5), (6) und (7) enthalten entweder eine Maleinsäureestergruppe oder eine Schwefelsäureestergruppe, die vorzugsweise in Form ihrer Alkalimetallsalze oder Ammoniumsalze vorliegen.

Von besonderem Interesse sind die sauren Phosphorsäureester der Anlagerungsprodukte der Formel (4), wobei die Phosphorsäureester zweckmässigerweise als Gemische der entsprechenden Salze eines Mono- und Diesters vorliegen.

Erfindungsgemässe Anlagerungsprodukte eignen sich für die verschiedensten Zwecke in der Textilapplikation, wie z.B. Vorbehandlung, Färben oder Ausrüstung. Nichtionogene unveresterte Produkte finden insbesondere Verwendung als Hilfsmittel beim Färben von polyamidhaltigen Fasermaterialien mit anionischen Farbstoffen oder Farbstoffgemischen. Die entsprechenden sauren Ester, besonders die Dicarbonsäure-Halbester, Schwefelsäureester und in erster Linie die Phosphorsäureester hingegen werden vorallem als Netzmittel, Entlüftungsmittel und Schaumdämpfungsmittel für wässrige Systeme, insbesondere beim Färben von natürlichem oder synthetischem Fasermaterial und in erster Linie beim Färben von Cellulose-Textilmaterialien, Polyesterfasern oder natürlichen oder synthetischen Polyamidfasermaterialien verwendet. Sowohl als nichtionogene Produkte als auch als saure Ester verbessern erfindungsgemässe Anlagerungsprodukte in den beschriebenen Formulierungen das Aufziehvermögen der Farbstoffe und beschleunigen dadurch die Diffusion der Farbstoffe in den Fasern.

Gegenstand der vorliegenden Erfindung ist demnach auch ein Verfahren zum Veredeln von natürlichem oder synthetischem Fasermaterial mit oder ohne entsprechende Farbstoffe, wobei die Veredelung in Gegenwart der erfindungsgemässen nichtionogenen und/oder anionischen Anlagerungsprodukte durchgeführt wird.

Die Einsatzmengen, in denen die erfindungsgemässen Anlagerungsprodukte den Veredelungsflotten, wie z.B. Färbeflotten oder Vor- oder Nachbehandlungsflotten zugesetzt werden, bewegen sich, je nach Substrat, zweckmässigerweise zwischen 0,5 bis 20 g, vorzugsweise 1 bis 10 g, pro Liter Flotte.

Vorteilhafte Anwendungsformen der erfindungsgemässen Anlagerungsprodukte bestehen darin, dass diese als nichtionogene und/oder anionische Verbindungen mit oder ohne Wasser in Kombination mit nichtionogenen oder anionischen Dispergatoren, Fettalkoholen, Fettsäureamiden, Alkylenbisfettsäureamiden, Alkenyldicarbonsäurealkylester, Metallstearaten, Siliconölen, z.B. Dialkylpolysiloxanen, oder auch Mineralölen oder Alkanolaminen in stabilen, netzwirkenden und/oder schaumdämpfenden Formulierungen eingesetzt werden. Derartige Zubereitungen zeichnen sich zudem dadurch aus, dass sie fähig sind, wässrige Systeme praktisch vollkommen zu entlüften. Es können somit sowohl in den Applikationsbädern als auch in den Substraten Lufteinschlüsse vermieden werden. Durch eine solche Entlüftung können Fleckenbildung bei Färbungen und Ausrüstungen ausgeschlossen werden.

Bevorzugt sind wässerige oder wasserfreie Zubereitungen, welche, bezogen auf die gesamte Zubereitung,

(A) 2 bis 50 Gew.-% eines sauren Esters des Anlagerungsproduktes der Formel (1) oder (4),

(B) 5 bis 50 Gew.-% eines nichtionogenen Tensides, vorzugsweise eines aliphatischen Monoalkohols mit 6 bis 22 Kohlenstoffatomen oder eines Anlagerungsproduktes von 2 bis 80 Mol Ethylenoxid an 1 Mol eines aliphatischen Monoalkohols mit 6 bis 22 Kohlenstoffatomen oder eines Polyoxyethylenderivates eines Sorbitanfettsäureesters oder eines Anlagerungsproduktes der Formel (1) oder (4) oder Gemische davon und mindestens eine der folgenden Komponenten:

(C) 1 bis 30 Gew.-% eines Silikonöls z.B. eines Dialkylpolysiloxans wie Dimethylpolysiloxan,

(D) 10 bis 60 Gew.-% eines Mineralöls z.B. Paraffinöl wie Shell Oil L 6189 oder Mineralöle Esso 301-312,

(E) 20 bis 45 Gew.-% eines Dialkylesters einer ethylenisch ungesättigten aliphatischen Dicarbonsäure, z.B. Maleinsäure-bis-2-ethylhexylester, Citraconsäure-bis-2-ethylhexylester

(F) 10 bis 70 Gew.-% eines Diffusionsbeschleunigers, insbesondere eines aliphatischen oder aromatischen Carbonsäureesters wie Milchsäurealkylester, Benzoesäurealkylester, Benzoesäurephenylester oder Benzoesäurebenzylester, oder auch eines Alkylbenzols, wie Trimethylbenzol, Ethylbenzol,

(G) 0,5 bis 5 Gew.-% eines Salzes einer $C_{10}$-$C_{24}$-Fettsäure und eines mehrwertigen Metalles, wie z.B. Magnesiumdistearat, Calciumdibehenat oder Aluminiumtristearat und

(H) 0,5 bis 3 Gew.-% eines $C_1$-$C_4$-Alkylendiamids einer Fettsäure mit 10 bis 24 Kohlenstoffatomen, wie z.B. Methylen-bis-stearinsäureamid, Ethylen-bis-stearinsäureamid und Ethylen-bis-behensäureamid enthalten.

Weitere bevorzugte wässrige Zubereitungen bestehen darin, dass sie bezogen auf die gesamte Zubereitung,

(a) 15 bis 50 Gew.% eines nichtionischen Anlagerungsproduktes von Styroloxid an einen Polyalkylenglykolether der Formel (1), besonders eines Styroloxid-Anlagerungsproduktes der Formel (4),

(b) 1 bis 5 Gew.% eines sauren Esters oder dessen Salzes, z.B. Alkalimetall- oder Ammoniumsalz, einer Verbindung der Formel

(8a) 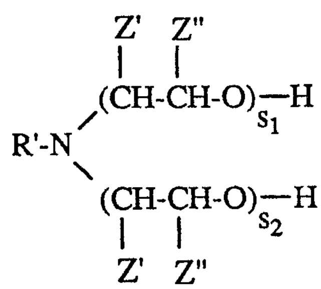

oder eines N-quaternierten Produktes des sauren Esters oder dessen Salzes, worin R′ einen Alkyl- oder Alkenylrest mit 12 bis 22 Kohlenstoffatomen, von Z′ und Z″ eines Wasserstoff, Methyl oder Phenyl und das andere Wasserstoff und $s_1$ und $s_2$ ganze Zahlen bedeuten, wobei die Summe $s_1$ + $S_2$ 2 bis 100 ist,

(c) 2 bis 10 Gew.% einer quaternären Ammoniumverbindung der Formel

(8b) 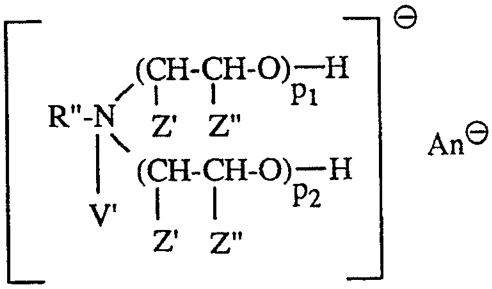

in der R″ einen Alkyl- oder Alkenylrest mit 12 bis 22 Kohlenstoffatomen, V′ einen gegebenenfalls substituierten Alkylrest, wie Methyl, Ethyl, Benzyl oder $-CH_2CONH_2$, von Z′ und Z″ eines Wasserstoff, Methyl oder Phenyl und das andere Wasserstoff, $An^{\ominus}$ ein Anion einer anorganischen oder organischen Säure und $P_1$ und $P_2$ je ganze Zahlen bedeuten, wobei die Summe von $p_1$ und $p_2$ bis 100 ist, und gegebenenfalls zusätzlich mindestens eine der folgenden Komponenten:

(d) 0,5 bis 25 Gew.% eines Polyalkylenglykolethers der Formel

R‴-O(Alkylen-O)$_q$H worin R‴ einen Alkyl- oder Alkenylrest mit 4 bis 22 Kohlenwasserstoffatomen bedeutet, "Alkylen" für den Ethylenrest oder Propylenrest steht und q 2 bis 85 ist, und

(e) 0,5 bis 5 Gew.% einer stickstoffhaltigen nichtionogenen Verbindung der Formel

(10) 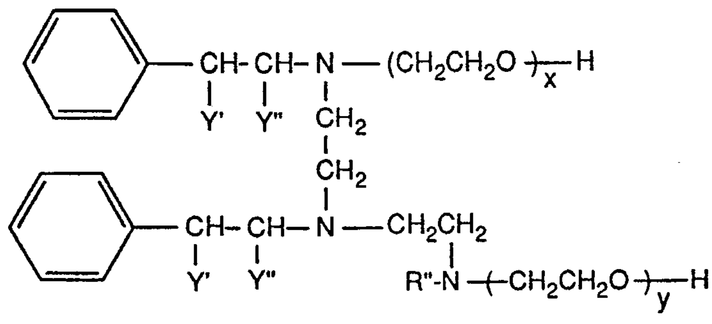

worin R″ einen Alkyl- oder Alkenylrest mit 12 bis 22 Kohlenstoffatomen, von Y′ und Y″ eines Phenyl und das andere Wasserstoff, und x und y ganze Zahlen bedeuten, wobei die Summe von x und y 80 bis 140 ist, enthält.

Nähere Angaben und bevorzugte Kombinationen und Anwendungsformen für Komponenten (b), (c), (d) und (e) können aus den Patentschriften DE-A-1 568 258, DE-A-1 619 385, EP-A-89004 und EP-A-312493 entnommen werden.

In den folgenden Herstellungs- und Anwendungsbeispielen beziehen sich die Prozentsätze, wenn nicht anders angegeben ist, auf das Gewicht; Teile sind Gewichtsteile. Die Mengen beziehen sich bei den Farbstoffen auf handelsübliche d.h. coupierte Ware und bei den Hilfsmitteln auf Reinsubstanz.

Herstellungsbeispiele

Beispiel 1:

a) 421,2 g Benzylalkohol werden zusammen mit 2,1 g Bortrifluorid-ethyletherat auf 50 °C erwarmt. In 60 Minuten lässt man 300 g Styroloxid zutropfen, wobei die Temperatur auf 85 °C ansteigt. Das Reaktionsgemisch wird noch 15 Minuten bei 85 °C nachgerührt, worauf der Ueberschuss an Benzylalkohol abdestilliert wird. Anschliessend wird im Hochvakuum fraktioniert destilliert. Man erhält 202 g einer farblosen Verbindung der Formel

(i) 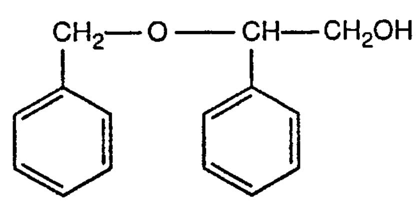

$Kp_{10}$-2: 119-122 °C OH-Zahl: 230

b) 243,5 g Benzyloxy-phenylethanol der Formel (i) werden in Gegenwart von 1 % Natriummethylat mit 88 g Ethylenoxid bei 140 °C und 2 bar Druck umgesetzt.

Man erhält eine Verbindung der Formel

(11) 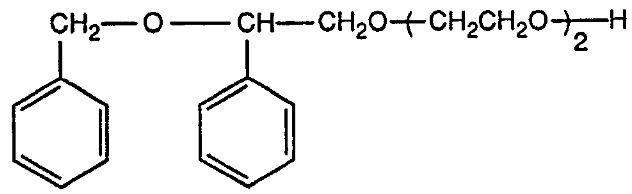

OH-Zahl:180

c) 51 g des Produktes der Formel (11) werden auf 60°C erwärmt und mit 17,2 g Harnstoff 15 Minuten gerührt. Dann gibt man 17,2 g Sulfaminsäure zu, erwärmt auf 70°C, rührt 1 Stunde bei 70°C, erwärmt auf 95°C und rührt weitere 2 Stunden bei 95°C. Nach Zugabe von 85,4 g Wasser erhält man eine 50%-ige klare Lösung der Verbindung der Formel

(12) 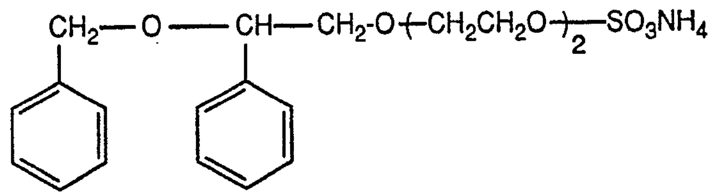

Beispiel 2: 51 g des Adduktes der Formel (11) werden zusammen mit 16,2 g Maleinsäureanhydrid auf 70°C erwärmt. Alsdann rührt man 1 Stunde bei 70°C, erhöht die Temperatur auf 90°C und rührt 3 weitere Stunden bei 90°C. Man erhält ein zähflüssiges klares Produkt, welches der Formel

(13) 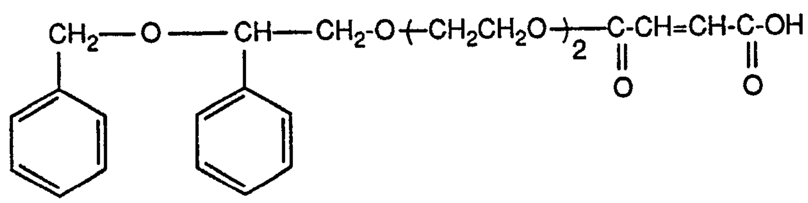

entspricht. Die Säurezahl beträgt 144.

Beispiel 3: 51 g des Adduktes der Formel (11) werden unter Rühren bei Raumtemperatur mit 7,8 g Phosphorpentoxid gemischt. Dabei steigt die Temperatur auf 72°C. Man rührt anschliessend 4 Stunden bei Raumtemperatur und erhält ein zähflüssiges Gemisch von Phosphorsäureestern der Formeln

(14a) 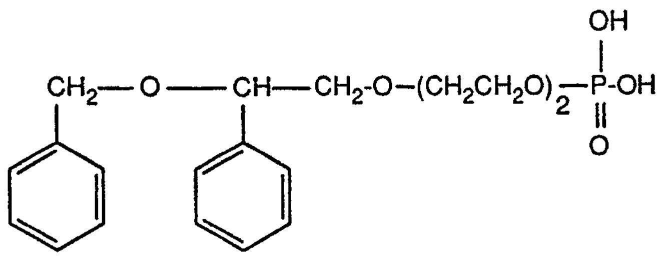 und

(14b) 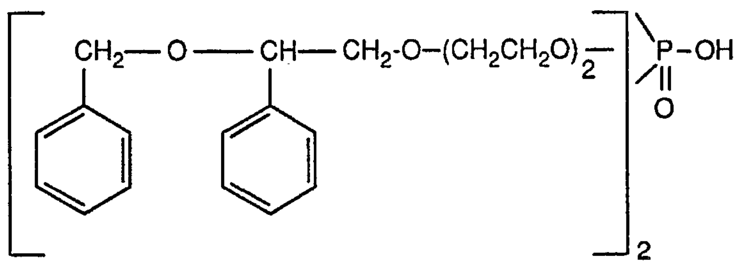

Auf ähnliche Art und Weise wie in den Beispielen 1 bis 3 beschrieben, werden unter Verwendung der entsprechenden Ausgangsprodukte die folgenden nichtionogenen und anionischen Anlagerungsprodukte hergestellt.

(15) 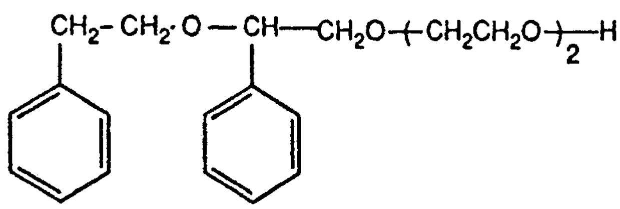

OH-Zahl: 188

(16) 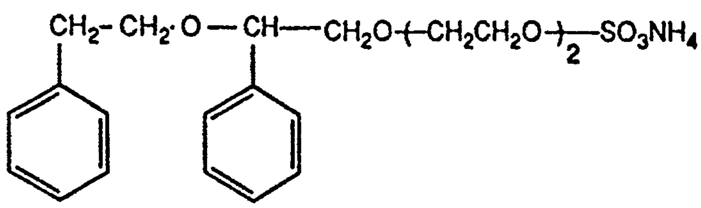

(17) 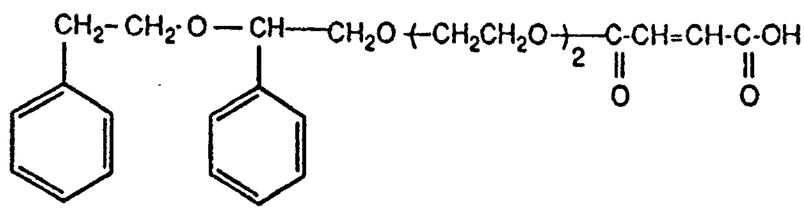

Säurezahl: 156

Gemisch eines Phosphorsäure-monoesters und -diesters der Formeln (18a) und (18b)

(18a) 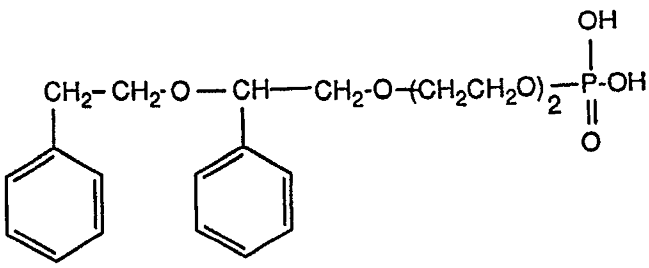 und

(18b) 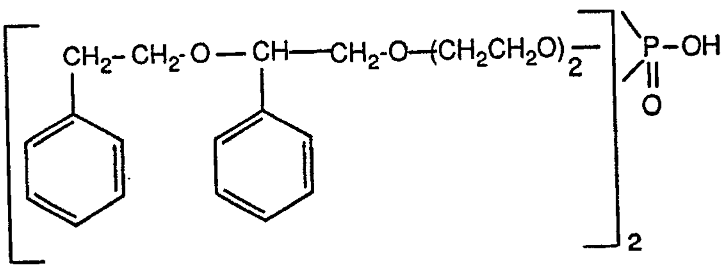

(19) 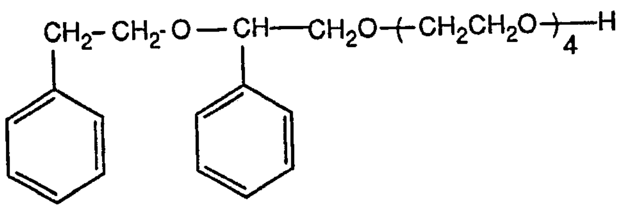

OH-Zahl: 159

(20) 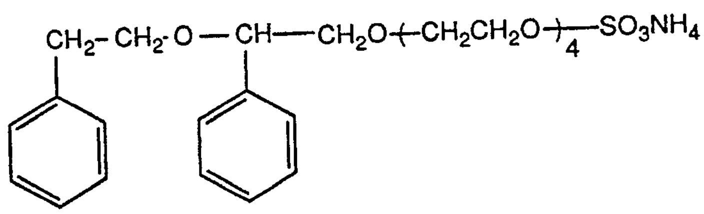

(21) 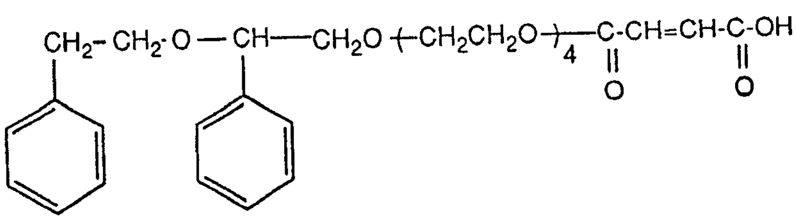

Säurezahl: 124

Gemisch eines Phosphorsäure-monoesters und -diesters der Formeln (22a) und (22b)

(22a) 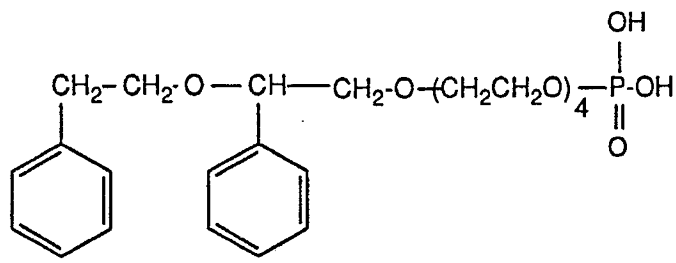 und

(22b) 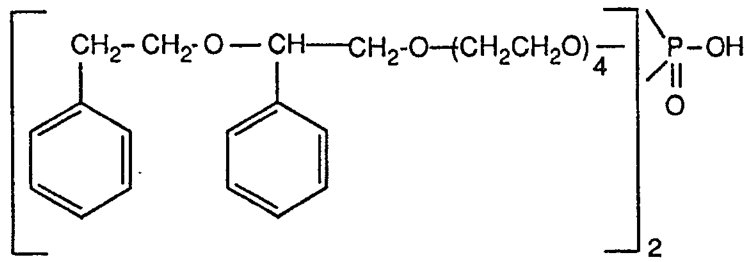

Anwendungsbeispiele

Beispiel 4: 100 g Wollserge werden in einem Zirkulationsapparat bei 40° C während 15 Minuten mit einer Flotte aus 1,1 Liter Wasser eingenetzt, welche folgende Zusätze enthält:

3,2 g Essigsäure 80 %

5,0 g Natriumsulfat kalz.

1 g des Anlagerungsproduktes der Formel (11).

Alsdann werden 5 g des Farbstoffes Acid Black 172 C.I. 15711 zugegeben. Nach 10 Minuten wird die Färbeflotte im Verlaufe von 45 Minuten auf 85° C erwärmt und das Färbegut unter ständiger Zirkulation 60 Minuten bei dieser Temperatur gehalten. Das Färbebad ist jetzt vollkommen erschöpft (Farbstoff auf der Faser). Anschliessend wird das Färbegut gespült und getrocknet. Man erhält ohne die konventionelle Erwärmung auf Kochtemperatur eine tiefe, egale schwarze Färbung der Wolle. Die Echtheiten entsprechen einer Färbung bei Kochtemperatur.

Beispiel 5: In einem Zirkulationsfärbeapparat werden 100 kg Kreuzspulen aus Wolle mit einem Materialträger eingefahren. Im Ansatzbehälter werden 1200 l Wasser auf 60° C erwärmt und darin 1200 g eines wässrigen Präparates gelöst, welches bezogen auf das Präparat,

12 % Silikonöl z.B. Dimethylpolysiloxan

15 % 2-Ethyl-n-hexanol

15 % Paraffinöl

2 % eines wasserlöslichen, oberflächenaktiven Siloxanoxyalkylencopolymerisates

8 % des anionischen Anlagerungsproduktes der Formel (13) und

2 % des nichtionogenen Anlagerungsproduktes der Formel (11)

enthält und mit Monoethanolamin auf pH 8 eingestellt ist. Dann wird die Flotte vom Ansatzbehälter durch das Material in den Färbeapparat gepumpt und anschliessend wechselseitig zirkuliert. Durch den Zusatz des Präparates tritt eine spontane Entlüftung des Färbesystems und damit eine gute Durchdringung des Färbegutes ein.

Hierauf gibt man der Flotte 2 kg des Farbstoffes der Formel

(101) 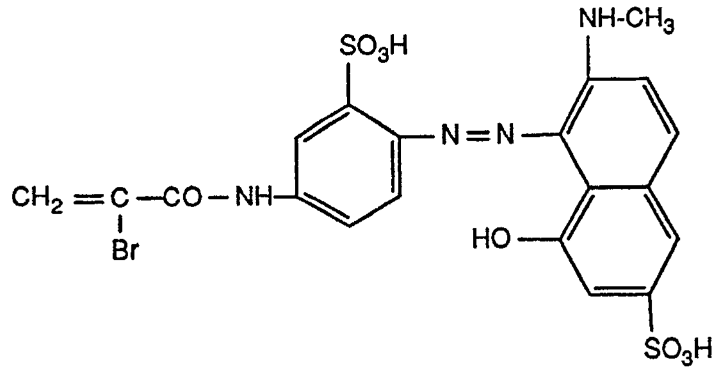

1 kg eines 1:1 Gemisches aus dem mit Chloracetamid quaternierten Anlagerungsproduktes von 7 Mol Ethylenoxid an 1 Mol eines $C_{16}$-$C_{18}$-Fettamingemisches und dem Ammoniumsalz des sauren Schwefelsäureesters des Anlagerungsproduktes von 7 Mol Ethylenoxid an 1 Mol eines $C_{16}$-$C_{18}$-Fettamingemisches (50%ige wässrige Zubereitung), erhitzt die Färbeflotte innerhalb 30 Minuten zum Sieden und färbt die Wolle 60 Minuten bei Siedetemperatur. Während der Färbung tritt praktisch keine Schaumentwicklung auf. Man erhält eine farbstarke und gleichmässige Durchfärbung der Kreuzspule.

Das in diesem Beispiel verwendete Präparat wird wie folgt hergestellt.

12 Teile Silikonöl werden in 15 Teilen 2-Ethyl-n-hexanol gelöst. Hierauf werden unter ständigem Rühren 15 Teile Paraffinöl, 2 Teile eines wasserlöslichen, oberflächenaktiven Siloxanoxyalkylencopolymerisates, 8 Teile des Maleinsäurehalbesters der Formel (13), 2 Teile des Anlagerungsproduktes der Formel (11), 47,6 Teile Wasser und 0,4 Teile Monoethanolamin zugesetzt und 30 Minuten weitergerührt. Man erhält eine lagerstabile Formulierung mit netzender, entlüftender und besonders schaumdämpfender Wirkung.

Beispiel 6: In einer Haspelkufe werden 100 kg Baumwollwirkware in 3000 l Wasser unter Zusatz von 3 kg eines wässrigen Präparates enthaltend, bezogen auf das Präparat,

7 % eines Silikonöls

11 % 2-Ethyl-n-hexanol und

11 % des anionischen Anlagerungsproduktes der Formel (12) genetzt.

Innerhalb von 30 Sekunden ist die Baumwolle vollständig genetzt und entlüftet. Hierauf werden der Flotte 2 kg eines Farbstoffes der Formel

(102) 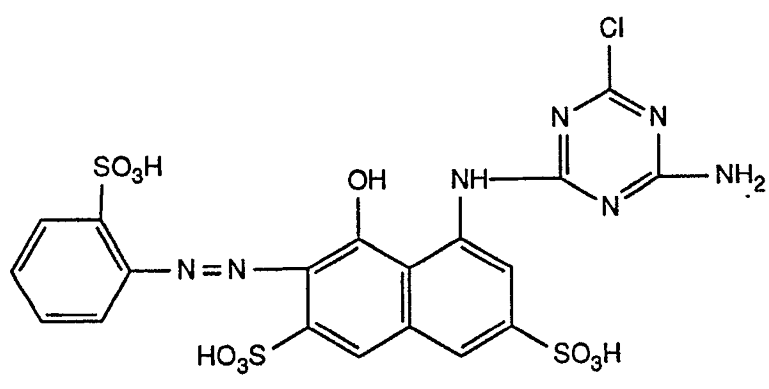

und die üblichen Chemikalien wie Elektrolyte und Alkalien zugesetzt, worauf die Ware zwei Stunden bei Siedetemperatur der Flotte gefärbt wird. Es tritt kein störendes Schäumen auf. Die gefärbte Ware ist gleichmässig und fleckenfrei durchgefärbt.

Das im Beispiel verwendete Präparat wird wie folgt hergestellt.

70 Teile Silikonöl werden zunächst in 110 Teilen 2-Ethyl-n-hexanol bei Raumtemperatur gelöst. Alsdann werden 110 Teile des Anlagerungsproduktes der Formel (12) und nach 30 Minuten 710 Teile Wasser zugegeben, worauf es noch 30 Minuten gerührt wird. Man erhält ein lagerstabiles Produkt mit ausgezeichneten netz- und entlüftenden Eigenschaften.

Beispiel 7: Auf einem Kurzflotten-Jet werden 100 kg Baumwolltricot in 600 Liter Wasser bei 40 °C eingenetzt. In die Flotte gibt man 36 kg Natriumchlorid, 5 kg des Farbstoffes der Formel (102) sowie 0,5 kg einer Zubereitung, enthaltend

186,75 g Mineralöl, (z.B. Shell Oil L 6189),
185 g Maleinsäure-bis-2-ethylhexylester,
10 g Magnesium-distearat
8,25 g N,N-Ethylenbisstearamid
55 g eines Polyoxyethylenderivates von Sorbitantristearat mit 20 Oxyethyleneinheiten z.B. Tween 65
55 g des anionischen Anlagerungsproduktes der Formel (12)

Man färbt die Ware während 45 Minuten bei 40°C. Dann erfolgt der Zusatz von 0,6kg kalziniertem Natriumcarbonat und nach weiteren 5 Minuten von 1,2 kg einer wässrigen 36%igen Natriumhydroxydlösung. Hierauf wird das Tricot während weiteren 40 Minuten gefärbt, anschliessend gespült und nachgewaschen. Man erhält eine echte, egale Rotfärbung des Tricots. Während des Färbeprozesses tritt kein Schäumen und keine Störung des Warenlaufes auf.

Die in diesem Beispiel verwendete Zubereitung wird wie folgt hergestellt.

186,75 g Mineralöl, 185 g Maleinsäure-bis-2-ethylhexylester, 10 g Magnesiumdistearat und 8,25 g N,N-Ethylen-bis-stearamid werden unter ständigem Rühren auf 110°C erwärmt bis eine klare Lösung eintritt. Man kühlt während 5 Minuten auf 45°C ab und verteilt in der Lösung 55 g eines Polyoxyethylenderivates von Sorbitantristearat mit 20 Oxyethyleneinheiten z.B. Tween 65 und 55 g des anionischen Anlagerungsproduktes der Formel (12). Man erhält eine stabile Formulierung, die insbesondere als Schaumdämpfungsmittel in alkalischen Flotten und bei hohen Scherkräften sehr wirksam ist.

Beispiel 8: 100 Teile eines Polyestergewebes werden in eine 60°C warme Färbeflotte gegeben, die 1300 Teile Wasser, 2 g/l Ammoniumsulfat, 2,5 Teile eines Farbstoffes der Formel

(103) 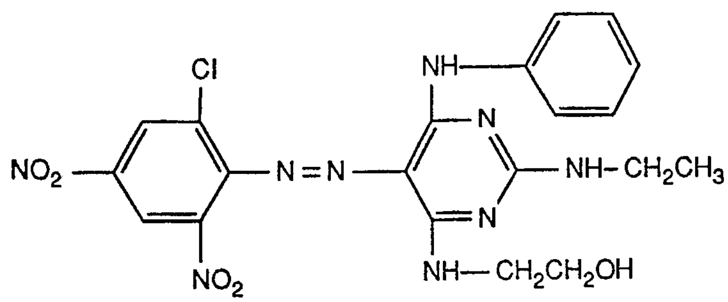

und 2 Teile einer Hilfsmittelformulierung bestehend aus
16 Teilen des anionischen Anlagerungsproduktes der Formel (13)
24 Teilen des Anlagerungsproduktes von 18 Mol Ethylenoxid an 1 Mol eines $C_{12}$-$C_{18}$-Fettalkoholgemisches und
60 Teilen Benzoesäurebenzylester
enthält und mit Ameisensäure auf pH 5 gestellt ist. Man steigert die Temperatur der Flotte in Verlauf von 30 Minuten auf 130°C und färbt 60 Minuten bei dieser Temperatur.

Danach wird die Flotte auf 70°C abgekühlt, abgelassen und die Ware gespült. Man erhält ohne die übliche reduktive Nachreinigung eine egale und reibechte rote Färbung mit einer hohen Farbstoffausbeute.

Die in diesem Beispiel verwendete Hilfsmittelformulierung wird wie folgt hergestellt:

In einem heizbaren Rührgefäss werden 60 Teile Benzoesäurebenzylester auf 60°C unter ständigem Rühren erwärmt und anschliessend 24 Teile des Anlagerungsproduktes von 18 Mol Ethylenoxid an 1 Mol eines $C_{12}$-$C_{18}$-Fettalkoholgemisches und 16 Teile des Anlagerungsproduktes der Formel (13) eingerührt. Man erhält nach Abkühlen auf Raumtemperatur eine lagerstabile Formulierung, die insbesondere beim Färben von Polyesterfasern geeignet ist.

Beispiel 9: 100 Teile eines Wickelkörpers aus texturierten Polyestergarnen werden in einen HT-Färbeapparat, welcher 800 Teile 40°C warmes Wasser, 2 Teile Ammoniumsulfat, 4 Teile eines Farbstoffes der Formel

(104) 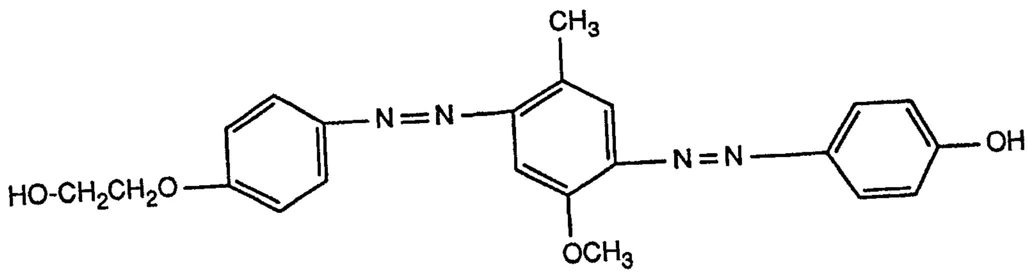

und 2 Teile der Hilfsmittelformulierung gemäss Beispiel 8 enthält und dessen Flotte mit Ameisensäure auf pH 5,5 gestellt ist, eingebracht. Danach wird die Temperatur der Flotte in Verlaufe von 40 Minuten auf 128 °C erhöht und die Ware 60 Minuten bei dieser Temperatur gefärbt. Während der Aufheizphase wird im Materialblock kein Anstieg des Differenzdruckes zwischen innen und aussen festgestellt. Anschliessend wird die Flotte auf 70 °C abgekühlt und das Substrat wie üblich reduktiv gereinigt, gespült und getrocknet. Man erhält eine farbstarke und egale orange Färbung, die sich durch gute Durchfärbung uind gute Echtheiten auszeichnet.

Beispiel 10: Ein Polyamid-(6,6)-Teppich (Schlingenflor) wird auf einem Foulard bei 25 °C mit einer Zubereitung, welche im Liter

1,2 g eines Farbstoffes der Formel

(105) 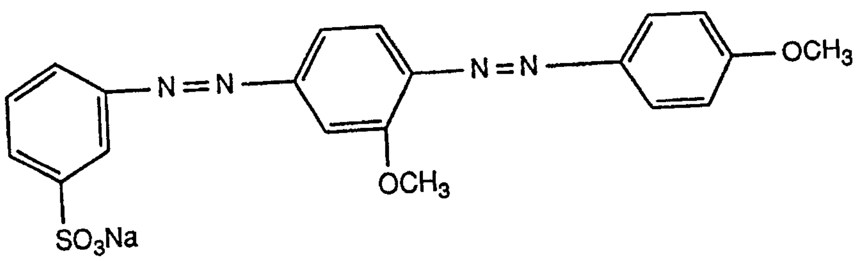

0,8 g eines Farbstoffes der Formel

(106) 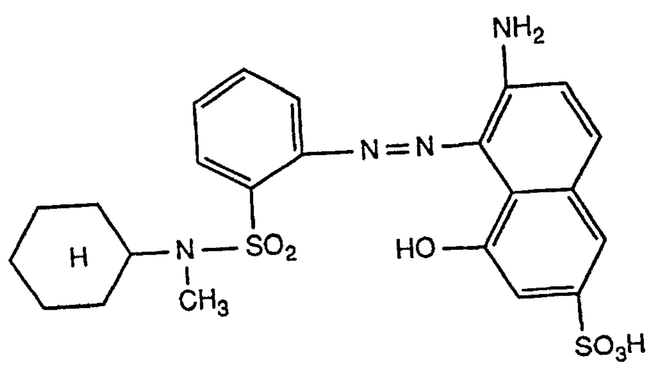

0,8 g eines Farbstoffes der Formel

(107) 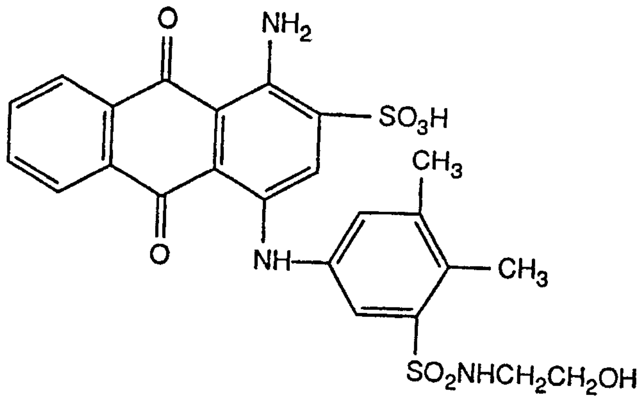

5 g des gemäss Beispiel 1 (c) hergestellten Anlagerungsproduktes der Formel (12)
2 g Verdickungsmittel und
1 g Natriumacetat
enthält und mit Essigsäure auf pH 5-5,5 eingestellt ist, imprägniert. Die Flottenaufnahme beträgt 320 %. Der imprägnierte Teppich wird mit Sattdampf im "Dog-house-steamer" bei 100 °C während 5 Minuten gedämpft. Anschliessend wird der Teppich mit kaltem Wasser gespült und getrocknet. Man erhält eine farbkräftige braune Färbung, ohne Grauschleier und mit dem erwarteten Echtheitsstandard.

Beispiel 11: 100 g Wollserge werden in einem Zirkulationsapparat z.B. Ahiba-Turbomat bei 40 °C während 15 Minuten mit einer Flotte aus 1,1 Liter Wasser eingenetzt, welche folgende Zusätze enthält:
3,2 g Essigsäure 80 %
5 g Natriumsulfat kalz.
2 g einer wässrigen Zubereitung enthaltend, bezogen auf diese Zubereitung, 30 Gew.% des gemäss Beispiel 1 hergestellten Styroloxidanlagerungsproduktes der Formel (11),
10 Gew.% des Hilfsmittelgemisches $A_1$ gemäss EP-A-0089004 und
20 Gew.% Triethylenglykolmonobutylether

Alsdann werden 4 g des Farbstoffes Acid Blaok 172 CI. 15711 zugegeben. Nach 10 Minuten wird die Färbeflotte im Verlauf von 50 Minuten auf 90 °C erwärmt und das Färbegut unter ständiger Zirkulation 60 Minuten bei dieser Temperatur gehalten. Anschliessend wird das Färbegut gespült und getrocknet.

Man erhält eine tiefe, egale und echte schwarze Färbung der Wolle. Das Färbebad ist zu 95 % erschöpft, obwohl bei 90 °C gefärbt wird. Die Echtheiten entsprechen einer konventionellen Färbung bei Kochtemperatur (98 °C).

## Ansprüche

1. Anlagerungsprodukte eines Alkylenoxids und Styroloxid an ein Arylalkanol, ihre saure Ester und deren Salze.

2. Anlagerungsprodukte gemäss Anspruch 1, dadurch gekennzeichnet, dass sie der Formel

(1) 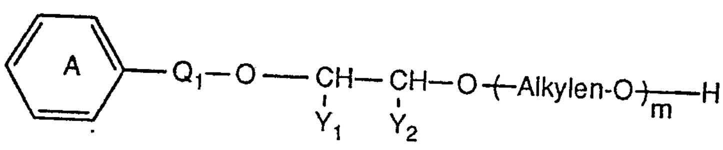

entsprechen, worin der Ring A einen unsubstituierten oder einen durch Halogen, Methyl oder $C_1$-$C_3$-Alkoxy substituierten Phenylrest,
$Q_1$ Alkylen mit 1 bis 4 Kohlenstoffatomen,
von $Y_1$ und $Y_2$ eines Phenyl und das andere Wasserstoff,
"Alkylen" einen Alkylenrest von 2 oder 3 Kohlenstoffatomen und
m eine Zahl von 1 bis 80 bedeuten.

3. Anlagerungsprodukte gemäss Anspruch 1, dadurch gekennzeichnet, dass sie der Formel

(2) 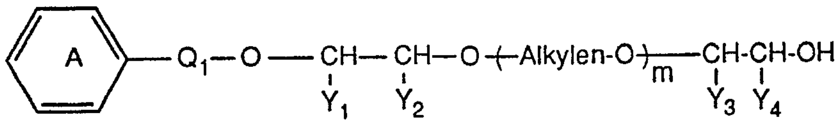

entsprechen, worin
der Ring A einen unsubstituierten oder einen durch Halogen, Methyl oder $C_1$-$C_3$-Alkoxy substituierten Phenylrest,
$Q_1$ Alkylen mit 1 bis 4 Kohlenstoffatomen in den Substituentenpaaren ($Y_1$ und $Y_2$) und ($Y_3$ und $Y_4$) ein Y Phenyl und das andere Y Wasserstoff,
"Alkylen" einen Alkylenrest von 2 oder 3 Kohlenstoffatomen und
m eine Zahl von 1 bis 80 bedeuten.

4. Anlagerungsprodukte gemäss Anspruch 1, dadurch gekennzeichnet, dass sie der Formel

(3) 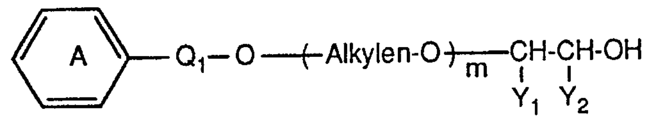

entsprechen, worin
der Ring A einen unsubstituierten oder einen durch Halogen, Methyl oder $C_1$-$C_3$-Alkoxy substituierten Phenylrest,
$Q_1$ Alkylen mit 1 bis 4 Kohlenstoffatomen,
von $Y_1$ und $Y_2$ eines Phenyl und das andere Wasserstoff,
"Alkylen" einen Alkylenrest von 2 oder 3 Kohlenstoffatomen und
m eine Zahl von 1 bis 80 bedeuten.

5. Anlagerungsprodukte gemäss einem der Ansprüche 2 bis 4, dadurch gekennzeichnet, dass "Alkylen" Ethylen bedeutet.

6. Anlagerungsprodukte gemäss einem der Ansprüche 2 bis 5, dadurch gekennzeichnet, dass

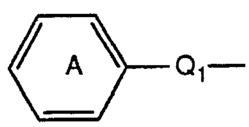

den Benzylrest bedeutet.

7. Anlagerungsprodukte gemäss einem der Ansprüche 2 bis 6, dadurch gekennzeichnet, dass m 2 bis 40, vorzugsweise 2 bis 10 bedeutet.

8. Anlagerungsprodukte gemäss Anspruch 2, dadurch gekennzeichnet, dass sie der Formel

(4) 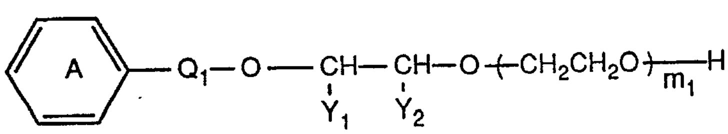

entsprechen, worin der Ring A einen unsubstituierten oder einen durch Halogen, Methyl oder Methoxy substituierten Phenylrest, von $Y_1$ und $Y_2$ eines Phenyl und das andere Wasserstoff und $m_1$ 2 bis 40, vorzugsweise 2 bis 10 bedeuten.

9. Saure Ester gemäss Anspruch 1, dadurch gekennzeichnet, dass sie der Formel

(5) 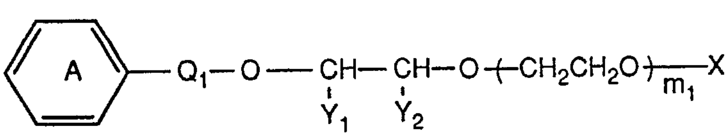

entsprechen, worin der Ring A einen unsubstituierten oder einen durch Halogen, Methyl oder Methoxy substituierten Phenylrest,

von $Y_1$ und $Y_2$ eines Phenyl und das andere Wasserstoff, X den Maleinsäure-, Sulfobernsteinsäure-, Schwefelsäure- oder Phosphorsäurerest und $m_1$ 2 bis 40, vorzugsweise 2 bis 10 bedeuten.

10. Saure Ester gemäss Anspruch 1 dadurch gekennzeichnet, dass sie der Formel

(6) 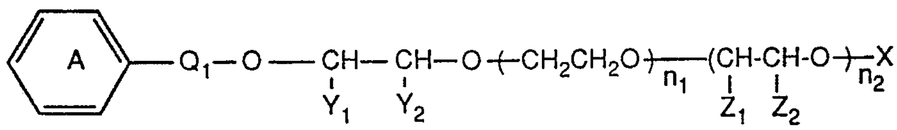

entsprechen, worin der Ring A einen unsubstituierten oder einen durch Halogen, Methyl oder Methoxy substituierten Phenylrest,

von $Y_1$ und $Y_2$ eines Phenyl und das andere Wasserstoff,

von $Z_1$ und $Z_2$ eines Methyl und das andere Wasserstoff,

X den Maleinsäure-, Sulfobernsteinsäure-, Schwefelsäure- oder Phosphorsäurerest und die Summe von $n_1 + n_2$ 2 bis 30, vorzugsweise 4 bis 18 bedeuten.

11. Saure Ester gemäss Anspruch 1 dadurch gekennzeichnet, dass sie der Formel

(7) 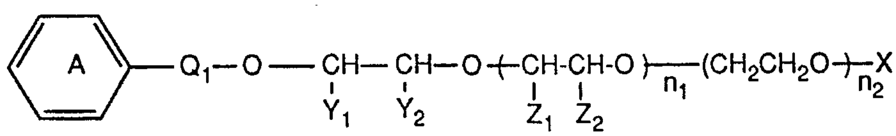

entsprechen, worin der Ring A einen unsubstituierten oder einen durch Halogen, Methyl oder Methoxy substituierten Phenylrest,

von $Y_1$ und $Y_2$ eines Phenyl und das andere Wasserstoff,

von $Z_1$ und $Z_2$ eines Methyl und das andere Wasserstoff,

X den Maleinsäure-, Sulfobernsteinsäure-, Schwefelsäure- oder Phosphorsäurerest und die Summe von $n_1 + n_2$ 2 bis 30, vorzugsweise 4 bis 18 bedeuten.

12. Wässerige oder wasserfreie Zubereitung, welche, bezogen auf die gesamte Zubereitung,

(A) 2 bis 50 Gew.-% eines sauren Esters einer Verbindung der Formel

(1) 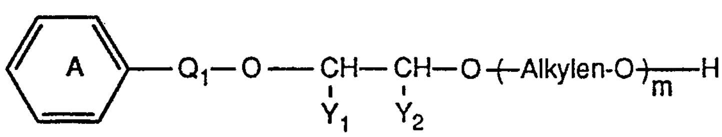 oder der Formel

(4) 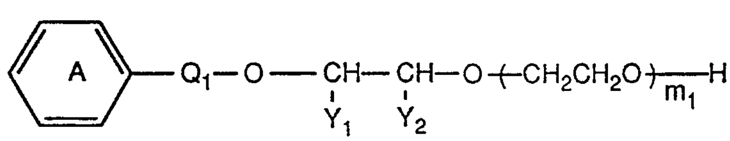

worin der Ring A einen unsubstituierten oder einen durch Halogen, Methyl oder $C_1$-$C_3$-Alkoxy substituierten Phenylrest,

$Q_1$ Alkylen mit 1 bis 4 Kohlenstoffatomen, besonders Methylen,

von $Y_1$ und $Y_2$ eines Phenyl und das andere Wasserstoff,

"Alkylen" einen Alkylenrest von 2 oder 3 Kohlenstoffatomen,

m eine Zahl von 1 bis 80 und $m_1$ eine Zahl von 2 bis 40 bedeuten,

(B) 5 bis 50 Gew.-% eines nichtionogenen Tensides und mindestens eine der folgenden Komponenten

(C) 1 bis 30 Gew.-% eines Silikonöls,

(D) 10 bis 60 Gew.-% eines Mineralöls,

(E) 20 bis 45 Gew.-% eines Dialkylesters einer ethylenisch ungesättigten aliphatischen Dicarbonsäure,

(F) 10 bis 70 Gew.-% eines Diffusionsbeschleunigers, insbesondere eines aliphatischen oder aromatischen Carbonsäureesters oder eines Alkylbenzols,

(G) 0,5 bis 5 Gew.-% eines Salzes einer $C_{10}$-$C_{24}$-Fettsäure und eines mehrwertigen Metalls und
(H) 0,5 bis 3 Gew.-% eines $C_1$-$C_4$-Alkylendiamids einer Fettsäure mit 10 bis 24 Kohlenstoffatomen
enthält.

13. Wässerige Zubereitung, welche bezogen auf die gesamte Zubereitung
(a) 15 bis 50 Gew.% eines nichtionogenen Styroloxidproduktes der Formel

(1) 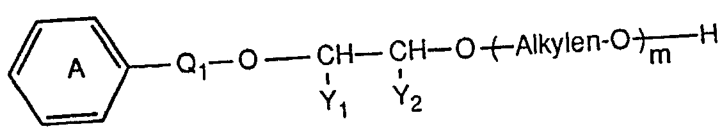 oder der Formel

(4) 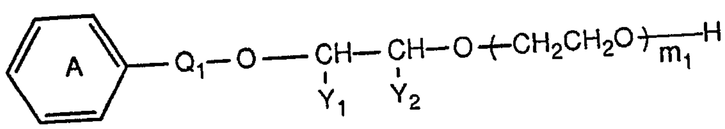

worin
der Ring A einen unsubstituierten oder einen durch Halogen, Methyl oder $C_1$-$C_3$-Alkoxy substituierten Phenylrest,
$Q_1$ Alkylen mit 1 bis 4 Kohlenstoffatomen, besonders Methylen,
von $Y_1$ und $Y_2$ eines Phenyl und das andere Wasserstoff,
"Alkylen", einen Alkylenrest von 2 oder 3 Kohlenstoffatomen,
m eine Zahl von 1 bis 80 und $m_1$ eine Zahl von 2 bis 40 bedeuten,
(b) 1 bis 5 Gew.% eines sauren Esters oder dessen Salzes, einer Verbindung der Formel

(8a) 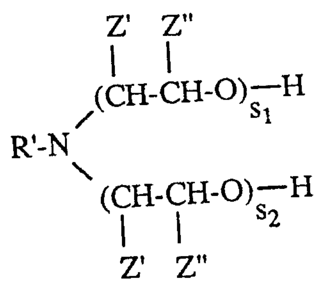

oder eines N-quaternierten Produktes des sauren Esters oder dessen Salzes, worin R′ einen Alkyl- oder Alkenylrest mit 12 bis 22 Kohlenstoffatomen, von Z′ und Z″ eines Wasserstoff, Methyl oder Phenyl und das andere Wasserstoff und $s_1$ und $s_2$ ganze Zahlen bedeuen, wobei die Summe $s_1 + s_2$ 2 bis 100 ist,
(c) 2 bis 10 Gew.% einer quaternären Ammoniumverbindung der Formel

(8b) 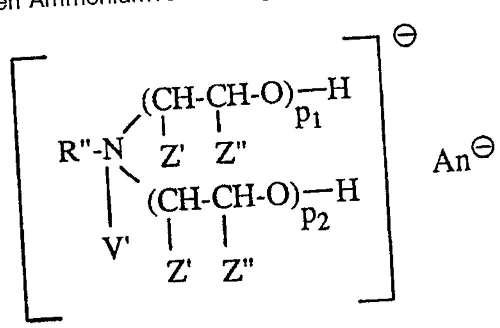

in der R″ einen Alkyl- oder Alkenylrest mit 12 bis 22 Kohlenstoffatomen, V′ einen gegebenenfalls substituierten Alkylrest, von Z′ und Z″ eines Wasserstoff, Methyl oder Phenyl und das andere Wasserstoff, $An^{\ominus}$ ein Anion einer anorganischen oder organischen Säure und $p_1$ und $p_2$ je ganze Zahlen bedeuten, wobei die Summe von $p_1$ und $p_2$ 2 bis 100 ist, und gegebenenfalls zusätzlich mindestens eine der folgenden Komponenten:

(d) 0,5 bis 25 Gew.% eines Polyalkylenglykolethers der Formel

$$(9)\ R'''-O-(\text{Alkylen-}O)_q-H$$

worin R‴ einen Alkyl- oder Alkenylrest mit 4 bis 22 Kohlenwasserstoffatomen bedeutet, "Alkylen" für den Ethylenrest oder Propylenrest steht und q 2 bis 85 ist, und

(e) 0,5 bis 5 Gew.% einer stickstoffhaltigen, nichtionogenen Verbindung der Formel

(10) 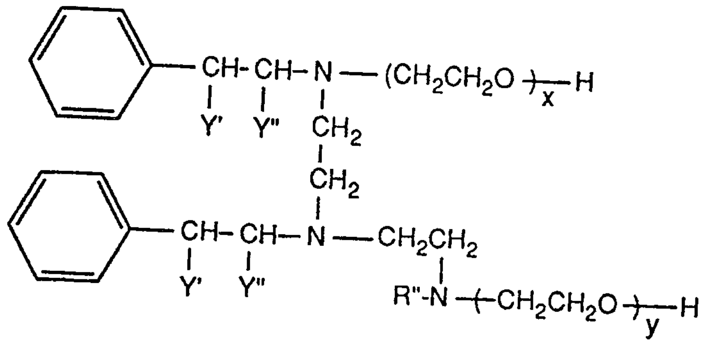

worin R″ einen Alkyl- oder Alkenylrest mit 12 bis 22 Kohlenstoffatomen, von Y′ und Y″ eines Phenyl und das andere Wasserstoff, und x und y ganze Zahlen bedeuten, wobei die Summe von x und y 80 bis 140 ist, enthält.

14. Verwendung der Verbindungen gemäss einem der Ansprüche 1 bis 11 oder der Zubereitung gemäss Anspruch 12 oder 13 beim Veredeln von natürlichem oder synthetischem Fasermaterial, insbesondere von natürlichen oder synthetischen Polyamidfasern, Polyesterfasern oder Cellulosefasern mit oder ohne entsprechenden Farbstoffen.

Europäisches Patentamt

# EUROPÄISCHER RECHERCHENBERICHT

Nummer der Anmeldung

EP 90 81 0713

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| X,P | EP-A-0 382 138 (CIBA-GEIGY AG) * Ansprüche * | 1-14 | C 08 G 65/32<br>C 08 G 65/26<br>D 06 P 1/613 |
| X,P | EP-A-0 378 048 (CIBA-GEIGY AG) * Ansprüche * | 1-14 | |
| Y | EP-A-0 173 879 (DAI-ICHI SEIYAKU CO., LTD) * Anspruch 1 * | 1-14 | |
| Y | EP-A-0 197 005 (CIBA-GEIGY AG) * Seite 3, Zeilen 1-10; Anspruch 1 * | 1-14 | |
| A | EP-A-0 197 001 (CIBA-GEIGY AG) * Ansprüche * | | |

RECHERCHIERTE SACHGEBIETE (Int. Cl.5)

C 08 G
D 06 P
D 06 M

Der vorliegende Recherchenbericht wurde für alle Patentansprüche erstellt

| Recherchenort | Abschlußdatum der Recherche | Prüfer |
|---|---|---|
| DEN HAAG | 26-11-1990 | PRAS J-L.C.N. |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPO FORM 1503 03.82 (P0403)